Europäisches Patentamt

European Patent Office

Office européen des brevets

(19)

(11) Publication number: **0 171 255**
A2

(12) # EUROPEAN PATENT APPLICATION

(21) Application number: 85305424.5

(22) Date of filing: 30.07.85

(51) Int. Cl.⁴: **A 61 F 5/445**

(30) Priority: 08.08.84 GB 8420127

(43) Date of publication of application: **12.02.86 Bulletin 86/7**

(84) Designated Contracting States: **AT BE CH DE FR GB IT LI LU NL SE**

(71) Applicant: **CRAIG MEDICAL PRODUCTS LIMITED, 204a London Road, East Grinstead Sussex RH19 1EY (GB)**

(72) Inventor: **Steer, Peter Leslie, Woodlands Rise, Kingscote East Grinstead Sussex (GB)**

(74) Representative: **Cook, Anthony John et al, D. YOUNG & CO. 10, Staple Inn, London, WC1V 7RD (GB)**

(54) Ostomy appliance.

(57) An ostomy appliance has interengaging coupling elements. To prevent discharged waste material from lodging in recesses or crevices in the coupling, a chute member is located inwardly of the stomal orifice of the coupling relative to the innermost wall of the coupling elements.

## OSTOMY APPLIANCE

This invention relates to an ostomy appliance.

Ostomy appliances are used by patients who have undergone abdominal surgery as a result of which an abdominal opening or stoma has been left in the body.

Such surgical techniques are well known and, by way of example, reference is made to colostomy operations (in which the large intestine is brought through the abdominal wall) and illeostomy operations (in which the large intestine is completely removed and the small intestine is brought through the abdominal wall). In each case, drainage or discharge from the digestive system of the patient takes place through the opening or stoma in the abdominal wall, and it is necessary to provide means to receive this drainage or discharge from the stoma which cannot be controlled by the patient.

One very successful commercially available arrangement is that known as the "SURGICARE SYSTEM 2" available from E. R. Squibb & Sons Limited. This has a first and second coupling element which connects to an ostomy bag or a pad or dressing on the patient. Reference may be made to U.S. Patent No. 4,460,363 entitled OSTOMY BAG which issued to Peter L. Steer and John V. Edwards on July 17, 1984.

For patients whose discharge is of a slurry-like nature, difficulties have sometimes been encountered in that the crannies and recesses often present in an ostomy appliance which includes interengaging coupling elements become filled with discharged waste material. This makes the task of changing a bag by uncoupling the old bag and coupling on a new bag an unpleasant one, especially for old or less dexterous persons.

According to the present invention, in an ostomy appliance having interengaging coupling elements, an additional chute member is included which is located inwardly of the stomal orifice of the coupling relative to the innermost wall of the coupling elements.

The chute member may be a molded plastic ring, e.g., of substantially cylindrical shape, and may be molded integrally with one coupling element. Preferably, however, the chute member is a separate molding. It may be attached to the pad by a plastic welding procedure, and it may have a flat outwardly-extending flange. The coupling element on the pad may be attached to the chute member flange by the same or by an additional plastic welding procedure. Heat welding or ultrasonic welding may be employed.

Brief Description of the Drawing

In the Drawing:

FIG. 1 is a central axial cross-section through one example of annular chute member according to the invention; and

FIG. 2 is a like cross-section through a coupling showing a chute member of FIG. 1 in its operative position.

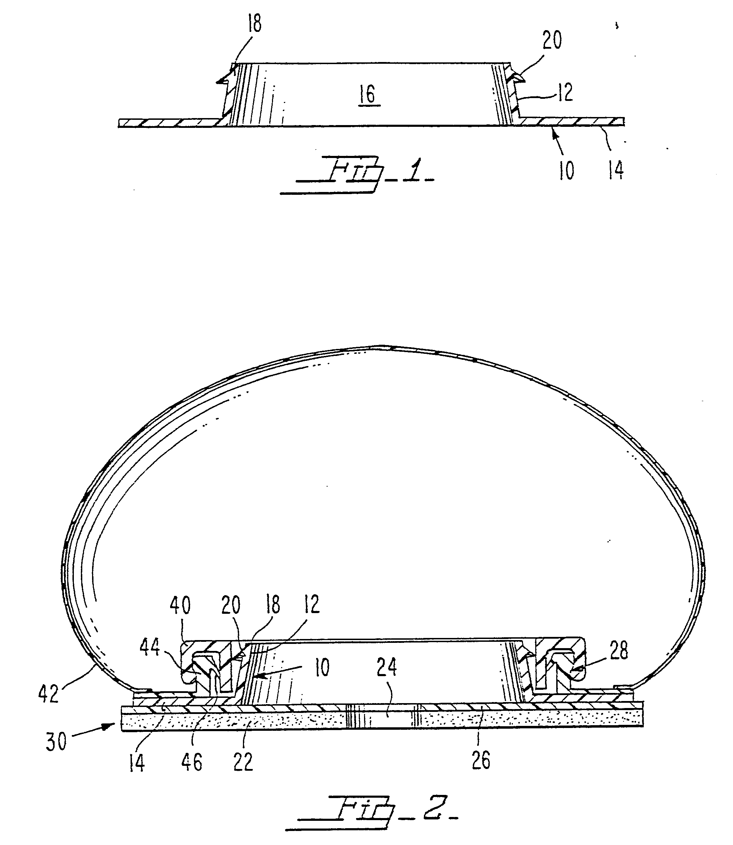

As indicated above, the present invention is directed to a chute member for an ostomy appliance, and to an ostomy appliance including such a member. One example of chute member is illustrated in cross-section in FIG. 1, from which it may be seen that the chute member 10 includes a chute ring 12 and a flat flange 14. The flange 14 is circular, but could be of any appropriate shape, for example, oval. Likewise, while the preferred form of chute ring 12 is circular, it could also be oval or square or rectangular with rounded corners. The chute ring 12 defines a stomal orifice 16 and can be regarded as substantially cylindrical. However, the preferred embodiment of chute ring 12 slopes inwardly in a direction away from the flange 14. That is, it is part-conical. It may for example be sloped at an angle of about 100 degrees to the plane of the disc 14.

On the outer surface of the chute ring 12, near its narrower end 18, there is located an annular flexible deflectible seal strip 20. This is intended to cooperate with an inner wall of a coupling element, as will be better understood from FIG. 2 herein.

FIG. 2 illustrates an ostomy bag coupling 30, including a pad 22 of suitable skin compatible material such as that sold under the trade mark "Stomahesive" by E. R. Squibb & Sons, and described in U.S. Patent No. 3,339,546 entitled BANDAGE FOR ADHERING TO MOIST SURFACES which issued on September 5, 1967. The pad 22 has a central hole 24 therein, to facilitate the user cutting a stomal orifice of suitable size to match their stoma, before applying the pad 22 in the convential manner. A thin plastic film 26 overlays the pad 22, in order to facilitate welding other plastic parts to the pad 22. A chute ring 10 in accordance with that disclosed in FIG. 1 is placed upon and welded to the flange 14 of the chute member 10, thereby completing the ostomy bag coupling 30.

In use, the narrower end of chute ring 10 is placed outside of the end of the patient's stoma, and the wall 12 of the chute ring 10 surrounds the stoma and ensures that no discharged material is likely to collect in parts of the ostomy bag coupling 30.

A bag side coupling element 40, for example in accordance with element 56 illustrated in U.S. Patent No. 4,460,363 entitled OSTOMY BAG which issued to Peter

-4-

L. Steer and John V. Edwards on July 17, 1984, cooperates with the ostomy bag coupling 30, and an ostomy bag 42, including a seal ring 44. Accordingly, when assembled, the inner wall of the element 40 is engaged by the seal strip 20, and a similar seal strip 46 on the seal ring 44, thereby assuring that discharged waste material will be kept well away from interengaging parts of the two coupling elements 30, 40.

While a particular embodiment of the invention has been particularly described and illustrated, it will be understood that modifications may be made without departing from the invention. Moreover, while the word "cylindrical" has been used in relation to chute ring 12, this word is not to be construed in a strictly geometrical sense but, rather, in accordance with the purpose and function of the part, namely to protect the remainder of the coupling elements from accumulation of discharged waste material.

-5-

What we claim is:

1. An improved ostomy appliance of the type comprising interengaging coupling elements, wherein the improvement comprises an additional chute member which is located within the stomal orifice of said coupling elements relative to the innermost wall of said coupling elements.

2. The improved ostomy appliance of Claim 1 in which said chute member comprised of a substantially cylindrical molded plastic ring.

3. The improved ostomy of Claim 2 in which said chute ring is comprised of a separate molding.

4. The improved ostomy of Claim 3 further comprising a pad for attachment to a wearer which carries one of said interengaging coupling elements, said other coupling element being secured or securable to an ostomy bag, said chute member being fixed to said pad by a plastic welding procedure.

5. The improved ostomy of Claim 4 in which said chute member has a radially outwardly-extending flange.

0171255

_Fig_1_

_Fig_2_